# EUROPEAN PATENT SPECIFICATION

(11) **EP 1 722 748 B1**
(45) Date of publication and mention of the grant of the patent: **02.07.2008**
(21) Application number: 05708325.5
(22) Date of filing: 19.02.2005
(51) Int. Cl.: A61K 8/18, A61Q 19/00

(54) **SKINCARE COMPOSITIONS CONTAINING SALICYLIC ACIDS**
HAUTFLEGEMITTEL DIE SALICYLSÄURE ENTHALTEN
COMPOSITIONS DE SOIN POUR LA PEAU CONTENANT DES ACIDES SALICYLIQUES

(30) Priority: 19.02.2004 GB 0403702; 31.08.2004 GB 0419260
(43) Date of publication of application: 22.11.2006
(73) Proprietor: Reckitt & Colman (Overseas) Limited, Slough Berkshire SL1 3UH (GB)
(72) Inventor: STRODTHOLZ, Iris, D-21465 Reinbek (DE); SCHMIDT, Timm, D-21465 Reinbek (DE); STRAHINJIC, Ivana, D-21465 Reinbek (DE)
(74) Representative: Cawdell, Karen Teresa
(86) International application number: PCT/GB2005/000503
(87) International publication number: WO 2005/079745

(56) References cited:
- EP-A- 0 696 451
- WO-A-95/16454
- WO-A-03/063816
- WO-A2-20/05025486
- CH-A5- 647 145
- CN-A- 1 088 087
- GB-A- 499 391
- GB-A- 1 050 756
- US-A- 4 147 782
- US-A- 4 608 370
- US-A- 5 693 318
- US-A- 5 736 582
- US-A1- 2002 031 556
- US-A1- 2002 172 719
- PATENT ABSTRACTS OF JAPAN vol. 008, no. 034 (C-210), 15 February 1984 (1984-02-15) & JP 58 198421 A (REIKO KOSAKA), 18 November 1983 (1983-11-18)

## Description

This invention relates to skincare compositions, in particular compositions effective in the treatment of acne vulgaris, and to the uses of such compositions in methods of treatment of the skin.

Acne vulgaris (acne) is a chronic inflammatory condition of the pilosebaceous units of the skin, which is particularly prevalent in adolescents. The condition generally causes the formation, on the skin, of comedones, red papules, pustules and sometimes cysts. This is unsightly and furthermore, if untreated, acne can lead to scarring of the skin. The major causes of acne are thought to be an increase in sebum production, an increased presence of *propionibacterium acne (P. acne),* blockage of the pilosebaceus duct and the production of inflammation.

Salicylic acid is known to be effective in the treatment of acne. It is a topical keratolytic agent that works by dissolving the intercellular cement that holds epithelial cells together. Salicylic acid is used in a variety of over-the-counter acne remedies.

In order to improve the efficacy of topical acne treatments, it is desired to formulate salicylic acid with one or more oil control agents such as sebum regulators which regulate the number of active glands or oil absorbing agents which remove excess oil from the skin. In order to ensure optimum performance, it is necessary to suspend the oil control agents in the skincare composition. However, a problem arises when producing such formulations as the ingredients necessary to suspend the oil control agents are less stable at the acidic pH of the salicylic acid compositions and satisfactory suspensions may not be formed. For example, using carbomer (trade name Carbopol, available from B.F. Goodrich) as the thickener in an aqueous carrier, it was found that combining salicylic acid with oil control agents such as talc, charcoal, rice starch and clay led to compositions which did not maintain the oil control agent in suspension for a satisfactory period. Similar results were also obtained with thickeners such as Acrylates/Palmeth-25 Acrylate copolymer (available from 3V Sigma S.P.A under the trade name Synthalen W2000) and xanthan gum (available from Kelco under the trade name Keltrol RD). In addition, it was found that incorporating sebum regulators such as Algae Extract, Burdock Extract, Watercress Extract and Orange extract into salicylic acid compositions led to compositions which were unable to form an acceptable hydro-lipid film on the skin. The formation of an acceptable hydro-lipid film on the skin is important to restore the equilibrium of all skin types by normalising the sebum flow through controlling the production of the sebum and regulating the number of effective glands.

WO 03063816 relates to topical personal care compositions containing an organic powder to improve the aesthetic feel of emulsion compositions comprising a partially and/or fully hydrolysed protein.

CH 647145 relates to a cosmetic formulation in the form of an emulsion comprising hydrogen peroxide and a constituent of a mammal's milk. The milk provides a very finely divided oily phase said to be useful with the peroxide to counteract drying of the skin.

EP 696451 relates to cosmetic formulation with anti-acne and/or keratolytic activity comprising a keratolytic compound complexed to a complexing molecule and a carrier therefor. The complex is said to provide improved stability and aesthetics for the formulation. Suitable complexing molecules are chosen from a wide variety of molecules including those which possess at least one free amino or hydroxy functional group.

WO 2005/025486 (published 24 March 2005) disclosed skincare compositions comprising hydrogen peroxide and salicylic acid. As an optional ingredient there may be included hydrolysed milk protein.

Surprisingly, it has now been found that skincare compositions comprising salicylic acid and hydrolysed milk protein have improved therapeutic efficacy in the treatment of acne. Said skincare compositions have both the ability to treat acne and establish a normal hydro-lipid film on the skin.

Hydrolysed milk protein is the hydrolysate of milk protein derived by acid, enzyme or other method of hydrolysis. Hydrolysed milk protein has previously been known for use to condition hair and skin. It may be used as a sebum regulator to restore the flow of sebum in dry skin and reduce excessive production of sebum in oily skin. It has not previously been employed as an active agent in the treatment of acne. However, in view of the results obtained with other oil control agents, it is surprising that hydrolysed milk protein in combination with salicylic acid has such a marked effect. For example, it has been shown that stable suspensions can be formed even at acid pH. Furthermore, trials have shown that even after a single application, both rinse-off and leave-on products cause the sebum level to be reduced for at least six hours. In addition, trials show a delay in the re-greasing of the skin and the achievement of significant reduction in the production of sebum even after a few days, for example three days, even with compositions that are rinsed off after application. With this combination, effective treatment of acne can be achieved as it is possible to restore the equilibrium of all skin types by normalising the sebum flow through controlling the production of the sebum and regulating the number of effective glands, even taking into account the over-drying and irritating effects of salicylic acid on the skin. Studies have also demonstrated that compositions containing a combination of salicylic acid and hydrolysed milk protein allow salicylic acid to penetrate deep into the pores, salicylic acid can even be detected in the pilosebaceous ducts. With repeated applications, salicylic acid can still be detected in the pores after up to eight hours, even when the product is rinsed off. Furthermore, the salicylic acid compositions are found to be well tolerated by the skin. This is particularly important as salicylic acid compositions can cause some degree of local skin peeling and discomfort such as burning and skin reddening.

Thus, according to a first aspect of the invention, there is provided a skincare composition with a pH in the range 2.5-6.0, suitable for topical application to the skin, the composition comprising 0.1-5% by weight salicylic acid or a salt thereof and hydrolysed milk protein, wherein the ratio of salicylic acid or salt thereof to hydrolysed milk protein is in the range from 2:1 to 15:1 parts by weight.

Salicylic acid is preferably incorporated into the composition according to the invention as the free acid. However, the pH of the composition may, and generally will, be such that the salicylic acid exists in the composition in dissociated form. As the composition may well contain cationic counterions, the salicylic acid may then be thought of as being present in salt form. Alternatively, the salicylic acid may be incorporated into the composition in salt form, eg as a salt with a Group I metal, such as sodium salicylate. As used herein, unless the context requires otherwise, any and all references to salicylic acid should be taken to encompass references to the acid and to dissociated forms and salts thereof.

The concentration of salicylic acid in the composition according to the invention is at least 0.1%, most preferably at least 0.5% and especially at least 1% by weight. The concentration of salicylic acid is preferably less than 5%, most preferably less than 4% and especially less than 3% by weight.

The concentration of salicylic acid may therefore fall in the range 0.1 % to 5%, and most preferably 0.5% to 4% and especially 1 to 3% by weight. A particularly preferred concentration of salicylic acid is 2% by weight.

The concentration of hydrolysed milk protein in the composition according to the invention is preferably at least 0.01 % by weight, more preferably at least 0.05% by weight, most preferably at least 0.08% by weight and especially at least 0.1 % by weight. The concentration of hydrolysed milk protein is preferably less than 2% and especially less than 1 % by weight. The concentration of hydrolysed milk protein may therefore fall within the range 0.05% to 3%, most preferably 0.08% to 2% and especially 0.1 to 1.0% by weight. A particularly preferred concentration of hydrolysed milk protein is 0.2% by weight.

In preferred compositions according to the present invention, the concentration of salicylic acid is in the range from 0.5 to 4% by weight, more preferably from 0.5 to 2% by weight and the concentration of hydrolysed milk protein is in the range from 0.08 to 2%, more preferably from 0.1 to 0.5 % by weight. In further compositions, the ratio of salicylic acid or salt thereof to hydrolysed milk protein is in the range from 2:1 to 15:1 parts by weight, most preferably from 5:1 to 12:1 parts by weight.

The composition is prepared with a pH in the range 2.5 to 6.0, particularly a pH in the range 2.5 to 4.0, eg about pH 3.0 or pH 3.5.

A composition according to the invention may comprise salicylic acid (or salt thereof) and hydrolysed milk protein as the sole active ingredients. However, a composition according to the invention may comprise one or more further topically active ingredients useful in skincare. Such active ingredients may include one or more of the following:
antimicrobial or antibacterial compounds, for example selected from the following:
   triclosan, neomycin, clindamycin, polymyxin, bacitracin, benzoyl peroxide, hydrogen peroxide, tetracylines such as doxycycline or minocycline, sulfa drugs such as sulfacetamide, penicillins, cephalosporins such as cephalexin, and quinolones such as lomefloxacin, olfoxacin or trovafloxacin;
   antiviral compounds, for example selected from acyclovir, tamvir, and penciclovir;
   antifungal compounds, for example selected from the following: farnesol, clotrimazole, ketoconazole, econazole, fluconazole, calcium or zinc undecylenate, undecylenic acid, butenafine hydrochloride, ciclopirox olaimine, miconazole nitrate, nystatin, sulconazole, and terbinafine hydrochloride;
   anti-inflammatory compounds, for example selected from the following: steroidal agents selected from hydrocortisone, fluocinolone acetonide, halcinonide, halobetasol propionate, clobetasol propionate, betamethasone dipropionate, betamethasone valerate, and triamcinolone acetonide, and non-steroidal anti-inflammatory agents selected from aspirin, ibuprofen, ketoprofen, naproxen, aloe vera gel, aloe vera, licorice extract, pilewort, Canadian willow root, zinc, and allantoin;
   anthelmintic compounds, for example metronidazole.

Particularly suitable antibacterial agents are peroxide antibacterial agents. A preferred peroxide antibacterial agent for inclusion in the composition is hydrogen peroxide. Alternatively, the composition may comprise a compound that, in use, is capable of generating hydrogen peroxide. An example of the latter class of compound is an adduct such as urea peroxide (carbamide peroxide).

In one preferred embodiment of the invention, the composition comprises a combination of salicylic acid, hydrolysed milk protein and hydrogen peroxide.

Where hydrogen peroxide is present in the composition according to the invention, the concentration of hydrogen peroxide is preferably at least 1% by weight. The concentration of hydrogen peroxide is preferably less than 5%, more preferably less than 3%, and most preferably less than 2%. The concentration of hydrogen peroxide may therefore fall within the range 1% to 5% by weight, more preferably 1% to 3%, and most preferably 1% to 2%.

The composition according to the invention may also comprise one or more ingredients which have a cooling effect on the skin, for example volatile ingredients, such as menthol.

The composition according to the invention may be formulated in numerous forms. However, the composition may often take the form of an aqueous or oily solution or dispersion or emulsion or a gel. An emulsion may be an oil-in-water emulsion or a water-in-oil emulsion.

The oil phase of water-in-oil or oil-in-water emulsions may comprise for example:
a) hydrocarbon oils such as paraffin or mineral oils;
b) waxes such as beeswax or paraffin wax;
c) natural oils such as sunflower oil, apricot kernel oil, shea butter or jojoba oil;
d) silicone oils such as dimethicone, cyclomethicone or cetyldimethicone;
e) fatty acid esters such as isopropyl palmitate, isopropyl myristate, dioctylmaleate, glyceryl oleate and cetostearyl isononanoate;
f) fatty alcohols such as cetyl alcohol or stearyl alcohol and mixtures thereof (eg cetearyl alcohol);
g) polypropylene glycol or polyethylene glycol ethers, eg PPG-14 butyl ether; or
h) mixtures thereof, for example, the blend of waxes available commercially under the trade name Cutina (Henkel).

Emulsifiers used may be any emulsifiers known in the art for use in water-in-oil or oil-in-water emulsions. Known cosmetically acceptable emulsifiers include:
a) sesquioleates such as sorbitan sesquioleate, available commercially for example under the trade name Arlacel 83 (ICI), or polyglyceryl-2-sesquioleate;
b) ethoxylated esters of derivatives of natural oils such as the polyethoxylated ester of hydrogenated castor oil available commercially for example under the trade name Arlacel 989 (ICI);
c) silicone emulsifiers such as silicone polyols available commercially for example under the trade name ABIL WS08 (Th. Goldschmidt AG);
d) anionic emulsifiers such as fatty acid soaps e.g. potassium stearate and fatty acid sulphates e.g. sodium cetostearyl sulphate available commercially under the trade name Dehydag (Henkel);
e) ethoxylated fatty alcohols, for example the emulsifiers available commercially under the trade name Brij (ICI);
f) sorbitan esters, for example the emulsifiers available commercially under the trade name Span (ICI);
g) ethoxylated sorbitan esters, for example the emulsifiers available commercially under the trade name Tween (ICI);
h) ethoxylated fatty acid esters such as ethoxylated stearates, for example the emulsifiers available commercially under the trade name Myrj (ICI);
i) ethoxylated mono-, di-, and tri-glycerides, for example the emulsifiers available commercially under the trade name Labrafil (Alfa Chem.);
j) non-ionic self-emulsifying waxes, for example the wax available commercially under the trade name Polawax (Croda);
k) ethoxylated fatty acids, for example, the emulsifiers available commercially under the trade name Tefose (Alfa Chem.);
l) methylglucose esters such as polyglycerol-3 methyl glucose distearate available commercially under the name Tegocare 450 ( Degussa Goldschmidt); or
m) mixtures thereof.

Gels provided according to the invention may be aqueous or non-aqueous. Aqueous gels are preferred. The gel will contain a gelling agents in order to give sufficient viscosity to the gel. A particularly suitable gelling agent is a copolymer of acryloyl dimethyl tauric acid (or a salt thereof), especially a copolymer of that monomer with another vinylic monomer. The salt may be a salt of a Group I alkali metal, but is more preferably an ammonium salt. Examples of suitable copolymer gelling agents are ammonium acryloyl dimethyl taurate / vinyl pyrrolidone copolymer, ammonium acryloyl dimethyl taurate / Beheneth-25 methacrylate copolymer, ammonium acryloyldimethyltaurate / vinyl formamide copolymer, These materials are available from Clariant GmbH in the range of products under the trade name Aristoflex.

A variety of thickening agents may also be used according to the nature of the liquid carrier and the viscosity required. Thickeners that are water-soluble or hydrophilic are preferred, and examples include acrylic acid polymers, eg those available commercially under the trade name Carbopol (B.F. Goodrich), modified celluloses, eg hydroxypropylmethylcellulose or hydroxyethylcellulose available commercially under the trade name Natrosol (Hercules), alkylgalactomanans available under the trade name N-Hance, xanthan gum, cetyl alcohol and sodium chloride.

The amount of gelling and/or thickening agent in the composition will each preferably lie in the range 0.1 to 5% w/w, more preferably 0.5 to 5% w/w. Typically, the amount of gelling and/or thickening agent will each be less than 3% w/w, eg about 1% w/w or about 2% w/w.

The composition according to the invention preferably has a viscosity of from about 50 mPa.s to about 20,000 mPa.s, more preferably from about 100 mPa.s to about 10,000 mPa.s. Viscosity may be measured using a Brookfield RVT viscometer equipped with a spindle 4 rotating at 10rpm after 2 minutes.

In many instances, it is preferred that the composition should comprise a chelating or sequestering agent, or other agent capable of complexation or other interaction with metal ions present in the composition. Such agents may improve the stability of the composition, and in particular may inhibit or prevent degradation of several ingredients (eg fragrance). Examples of chelating or sequestering agents include ethylenediamine tetraacetic acid and its salts, notably the dipotassium and especially the disodium salt.

In the case of solutions or dispersions, and gels, the composition will generally contain a solvent system or other continuous liquid phase. Such a system is preferably aqueous. However, mixed solvent systems may often be used with advantage. Such a mixed solvent system most preferably comprises water, in admixture with a co-solvent, most preferably a lower (eg C₁₋₆) alcohol, in particular ethanol and t-butyl alcohol.

Preferred aqueous systems comprise water in an amount of at least 50% by weight, more preferably at least 60% by weight, most preferably at least 70% by weight and especially at least 80% by weight. The upper limit of water will depend on the amounts of other ingredients incorporated in the composition so that the water may form the remainder of the composition up to 100% of the composition. A typical maximum value is less than 90% by weight, for example 80% by weight or 85% by weight.

The composition most preferably comprises in excess of 5% w/w of the cosolvent, and may comprise in excess of 10% w/w, in excess of 20% w/w, or in excess of 30% w/w of the cosolvent. The amount of cosolvent present in the composition preferably does not exceed 50% w/w. The amount of cosolvent thus preferably lies in the range 5% to 50% w/w, more preferably 10% to 50% w/w. In general, higher proportions of cosolvent may be required in compositions containing higher proportions of ingredients (eg topically active ingredients, as discussed above) that are of low solubility in water. Where such ingredients are absent, of their concentration is relatively low, the proportion of cosolvent may also be somewhat lower than in other embodiments, eg up to 20% w/w.

The composition may additionally comprise other components which will be well known to those skilled in the art. These include, for example:
a) Emollients - ingredients that help to maintain the soft, smooth and pliable appearance of skin. Such ingredients may function by their ability to remain on the surface of the skin or in the stratum corneum, and to act as lubricants, reducing or preventing flaking of the skin and improving the skin's appearance. Examples of emollients are isopropyl myristate, triglycerides of fatty acids eg lauric triglyceride or capric/caprylic triglyceride, such as the triglyceride available commercially under the trade name Miglyol 810 (Huls UK), and the polypropylene glycol ether of stearyl alcohol known as PPF-15 Stearyl Ether. Particularly preferred emollients are polysiloxane compounds, in particular those known as cyclomethicone, ie cyclic dimethyl polysiloxane compounds that conform to the formula:

   -(Si(CH₃)₂)ₙ-

   in which n has a value between 3 and 7.
b) Humectants or Moisturisers - ingredients intended to increase the water content of the top layers of the skin. Examples of such ingredients are glycerin, 1,3-butylene glycol and propylene glycol.
c) Surfactants - Surfactants may be used in compositions according to the invention as solubilisers, or as cleansing agents or foam boosters. Many different classes of surfactant may be suitable for inclusion in the composition according to the invention, and these will be readily apparent to those skilled in the art. Examples of suitable surfactants include polyethylene glycol ethers of alcohols such as isocetyl alcohol (eg Isoceteth-20), isostearyl alcohol (eg Isosteareth-20), cetyl alcohol (eg Ceteth-20), oleyl alcohol (eg Oleth-20) and cetearyl alcohol (eg Ceteareth-20). A particularly preferred surfactant for use in the invention is lsoceteth-20.
d) Emulsion stabilising salts such as sodium chloride, sodium citrate or magnesium sulphate.
e) Preservatives - ingredients which prevent or retard microbial growth and thus protect the composition from spoilage. Examples of preservatives include such as propylparaben, bronopol, sodium dehydroacetate, polyhexamethylenebiguanide hydrochloride, isothiazolone and diazolidinylurea.
f) Chelating agents or sequestering agents (sequestrants) - ingredients that have the ability to complex with and inactivate metallic ions in order to prevent their adverse effects on the stability or appearance of the composition, as described above. Examples of chelating agents are ethylenediamine tetraacetic acid and its salts, notably the dipotassium and especially the disodium or tetrasodium salt.
g) Abrasives - ingredients used to assist in the removal of unwanted tissue or foreign materials from the skin during application of the composition. Abrasives commonly comprise fine solid particles. One example of a suitable abrasive is polyethylene beads.
h) pH adjusters - Ingredients used to control the pH of the composition. Examples of pH adjusters are inorganic salts such as sodium hydroxide, and organic bases such as triethanolamine.
i) Conditioning agents, for example distearyldimonium chloride.
j) Perfumes and colourings.

The composition according to the invention may be applied and left on the skin to have the desired therapeutic effect or it may be applied and then rinsed off, for example with water. The composition may be applied with the aid of a fibrous material, for example a pad or a wipe.

According to another aspect of the invention, there is provided an article comprising a fibrous substrate impregnated with a skincare composition with a pH in the range 2.5-6.0, suitable for topical application to the skin, the composition comprising 0.1-5% by weight salicylic acid or a salt thereof and hydrolysed milk protein, wherein the ratio of salicylic acid or salt thereof to hydrolysed milk protein is in the range from 2:1 to 15:1 parts by weight.
The fibrous material may be used to apply the composition onto the skin.

Preferably, said fibrous material is impregnated with the skincare composition according to the inventionin an amount in the range from 10 to 30% by weight, preferably from 15 to 25% by weight and most preferably from 18 to 22% by weight of the fibrous material. Suitable fibrous materials include cellulose or cotton fibres or a mixture thereof. The fibrous material may be impregnated with the composition as a wet wipe which is arranged for immediate use to apply the skincare composition of the present invention to the skin of the user. Alternatively, the fibrous material may be impregnated with the skincare composition and dried to form a dry wipe which requires to be wetted, for example with water, before it can be used.

In a yet further aspect of the invention, there is provided the use of hydrolysed milk protein in the preparation of a composition comprising 0.1-5% by weight salicylic acid or a salt thereof and having a pH in the range 2.5-6.0, wherein the ratio of salicylic acid or salt thereof to hydrolysed milk protein is in the range from 2:1 to 15:1 parts by weight for the prophylactic or remedial medical treatment of acne by topical application of the composition to the skin.

It will be appreciated that the method according to this aspect of the invention may be a therapeutic method, but will often be a primarily cosmetic method, the objective of which is to reduce or eliminate externally visible, and often unsightly, symptoms of acne vulgaris.

The invention will now be described in greater detail, by way of illustration only, with reference to the following Examples. In the Examples the hydrolysed milk protein is obtained from Sederma, France; the spunlace is obtained from Jacob Holm Industries (France) under the trade name Standard JH50gsm Spunlace JH501074L2.

### Example 1

### Oil Control Cream Wash

| Ingredients | %w/w |
|---|---|
| Sodium cocoyl isethionate | 16.40 |
| Cetyl alcohol | 10.00 |
| Laureth-3 | 5.00 |
| Glycerin | 3.00 |
| Salicylic acid | 2.00 |
| Sodium hydroxide | 0.36 |
| Hydrolysed Milk Protein | 0.20 |
| Parfum | 0.20 |
| Disodium EDTA | 0.01 |
| Aqua | to 100% |

### Method

The cetyl alcohol and laureth-3 were heated to 60°C to form the oil phase.
The remainder of the ingredients were mixed to form the aqueous phase.
The oil phase was added to the aqueous phase. The composition was cooled down to room temperature with stirring to form a uniform composition.

### Example 2

### Scrub Wipe

| Ingredients | %w/w |
|---|---|
| PPG-14 butyl ether | 8.00 |
| Cetearyl isononoate | 2.25 |
| Salicylic acid | 2.00 |
| Ceteareth-20 | 1.13 |
| Cetearyl alcohol | 1.13 |
| Glyceryl Stearate | 0.45 |
| Glycerin | 0.45 |
| Hydrolysed Milk Protein | 0.20 |
| Hydrogen peroxide (active 35%) | 1.50 |
| Menthol | 0.10 |
| Disodium EDTA | 0.10 |
| Cetyl palmitate | 0.15 |
| Ceteareth palmitate | 0.15 |
| Parfum | 0.10 |
| Aqua | to 100% |

### Method

All the ingredients, apart from hydrolysed milk protein, hydrogen peroxide, menthol and parfum, were mixed and heated to 90°C. The mixture was cooled down to room temperature with stirring. The remaining ingredients were stirred into the mixture to form a uniform composition.

The mixture was impregnated into a wipe material available as Standard Spunlace , width 895mm-1074mm, thickness 0.55mm, absorption capacity 1091%, basic weight 50.7g/m2,using 140ml lotion to 32 wipes.

### Example 3

### Gel Wash

| Ingredients | %w/w |
|---|---|
| Laureth sulphate | 11.9 |
| Coco glucoside | 4.0 |
| Glycerin | 3.0 |
| Salicylic acid | 2.0 |
| Sodium chloride | 1.6 |
| Hydrolysed Milk Protein | 0.2 |
| Parfum | 0.2 |
| Menthol | 0.1 |
| Aqua | to 100% |

### Method

All the ingredients were mixed at room temperature to form a uniform composition.

### Example 4

### Lotion for Impregnated Pads

| Ingredients | %w/w |
|---|---|
| Alcohol(99.9%) and t-Butylalcohol(0.1 %) | 37.00 |
| Isoceteth-20 | 3.00 |
| Salicylic acid | 2.00 |
| Hydrogen Peroxide (active 35%) | 4.29 |
| Hydrolysed Milk Protein (mixed with propylene glycol and water) | 0.20 |
| Sodium hydroxide (30%) | 0.20 |
| Parfum | 0.10 |
| Disodium EDTA | 0.005 |
| Aqua | to 100% |

### Method

All the ingredients were mixed at room temperature to form a uniform composition. The composition was impregnated into a non-woven pad consisting of rayon and polypropylene using approximately 100ml lotion for 65 pads.

### Example 5

### Cream Scrub

| Ingredients | %w/w |
|---|---|
| Stearyl alcohol | 3.00 |
| Cetyl alcohol | 1.00 |
| Salicylic acid | 2.00 |
| Glycerin | 3.00 |
| Xanthan gum | 0.20 |
| Steareth-21 | 0.50 |
| Steareth-2 | 0.25 |
| Distearyldimoniumchloride | 1.50 |
| Behenyl alcohol | 0.42 |
| PPG-15 Stearyl ether (99.9%) and BHT (0.1 %) | 4.00 |
| Mixture of Cetyl betaine (30%), | |
| sodium chloride (7%), | |
| alcohol (10%), water (10%) | 6.70 |
| Sodium lauryl sulphate | 3.60 |
| Polyethylene beads | 4.00 |
| Hydrolysed Milk Protein (mixed with propylene glycol and water) | 0.20 |
| Synthetic wax and mica beads | 0.35 |
| Parfum | 0.20 |
| Disodium EDTA | 0.01 |
| Aqua | to 100% |

### Method

The stearyl alcohol, cetyl alcohol, behenyl alcohol, steareth-21, steareth-2, distearyldimoniumchloride, PPG-15 stearyl ether and BHT were heated to 60°C to form the oil phase. The remainder of the ingredients were mixed to form the aqueous phase. The heated oil phase was added to the aqueous phase. The composition was cooled down to room temperature with stirring.

### Example 6

### Gel Lotion

| Ingredients | % w/w |
|---|---|
| Alcohol (99.9%) + t-butylalcohol (0.1 %) | 11.5 |
| Glycerin | 0.50 |
| Isoceteth-20 | 1.00 |
| Salicylic acid | 0.50 |
| Hydrogen peroxide (35%) | 4.29 |
| Ammonium acryloyldimethyltaurate/ vinyl pyrrolidone copolymer | 1.50 |
| Hydrolyzed Milk Protein | 0.20 |
| Sodium hydroxide (30%) | 0.40 |
| Parfum | 0.20 |
| Disodium EDTA | 0.005 |
| Aqua | to 100% |

### Method

The salicylic acid was mixed into the alcohol/t-butylalcohol. When the salicylic acid was fully dissolved, the water, glycerin and disodium EDTA were mixed in. The ammonium acryloyldimethyltaurate / vinyl pyrrolidone copolymer was added with continuous homogenisation, followed by the isoceteth-20, hydrogen peroxide, hydrolysed milk peptide and parfum in the water. The pH was adjusted to pH 3 with sodium hydroxide (30%).

### Example 7

### Lotion

| Ingredients | % w/w |
|---|---|
| Sorbitol (70%) | 0.50 |
| Denatured ethanol | 37.00 |
| Glycerin | 1.50 |
| Isoceteth-20 | 2.00 |
| Salicylic acid | 2.00 |
| Hydrolyzed Milk Protein | 0.20 |
| Sodium hydroxide (30%) | 0.38 |
| Parfum | 0.10 |
| Aqua | to 100% |

### Method

The salicylic acid, isoceteth-20 and parfum were dissolved in the ethanol to form the oil phase. The remaining ingredients were mixed with the water to form the aqueous phase. The oil and aqueous phases were mixed to form a uniform composition.

### Example 8

### Lotion

| Ingredients | % w/w |
|---|---|
| Sorbitol (70%) | 0.50 |
| Denatured ethanol | 37.00 |
| Hydrogen Peroxide | 4.29 |
| Isoceteth-20 | 3.00 |
| Salicylic acid | 2.00 |
| Hydrolyzed Milk Protein | 0.20 |
| Sodium hydroxide (30%) | 0.20 |
| Parfum | 0.10 |
| Disodium EDTA | 0.005 |
| Aqua | to 100% |

### Method

The salicylic acid, isoceteth-20 and parfum were dissolved in the ethanol to form the oil phase. The remaining ingredients were mixed with the water to form the aqueous phase. The oil and aqueous phases were mixed to form a uniform composition.

### Example 9

### Clinical Trial To Determine The Influence Of Cosmetic Products Containing 2% Salicylic Acid And 0.2% Hydrolysed Milk Protein On The Sebum Level Of The Skin

### Protocol

20 volunteers were included in a sebumetry study in order to evaluate the influence of several types of cosmetic formulations containing 2% salicylic acid and 0.2% HMP on the sebum level of the skin.
On the back of the volunteers, 7 areas of 5x5 cm were delimited in order to test 5 products : 3 rinse-off products (a gel wash (Example 3), a cream wash (Example 1), a cream scrub (Example 5)) and 2 leave-on products (impregnated pads (Example 4) and impregnated wipes (Example 2)).
An untreated area (used for the comparison versus leave on products) and an area washed by water and then dried (used for the comparison versus rinse-off products) was used as control. A randomisation step was used between the 7 areas on subjects in order to eliminate variations of sebum content linked with the localisation on the back.

Before any application, the sebum level was measured using a sebumeter^{®} SM810 (Courage and Khazaka) to define baseline sebum content on each area. Other measurements were performed 2, 4 and 6 hours after the standardised application of each product on each area.

### Results

The results are shown in Table 1 below.

**Table 1**

| | 0 hours | 2 hours | 4 hours | 6 hours |
|---|---|---|---|---|
| Untreated control | 147.8 | 153.3 | 142.7 | 141.5 |
| Gel wash | 152 | 36.3* | 45.2** | 74** |
| Cream wash | 151.9 | 33.7* | 45.7** | 72.5** |
| Cream scrub | 153.8 | 40.6* | 42.9** | 72.6** |
| Pad | 148.6 | 39* | 45.8** | 84.1 |
| Wipes | 143.7 | 45.5* | 49.3* | 78.8** |
| Water control | 156.8 | 42.5* | 114 | 140.9 |

| | | | | |
|---|---|---|---|---|
| *p<0.05 versus untreated value **p<0.05 versus untreated and wash only value | | | | |

The control area only treated with water showed statistically significant reduction only after 2 hours. Four and 6 hours after the treatment the level of sebum was not different to the untreated control.

The rinse off products (gel wash, cream wash and cream scrub) all demonstrated a statistically significant reduction of the sebum level versus water control area at two, four and six hours.

For the leave-on products, statistically significant reductions of the sebum level versus the untreated control area were found after 2, 4 and 6 hours for the impregnated wipe. Statistically significant reductions of the sebum level versus the untreated control area were found after 2 and 4 hours for the impregnated pad. The sebum quantity after 6 hours on the area treated by the pad, although being smaller, was not statistically different from the untreated area.

### Conclusion

Pads showed statistically significant reduction of the sebum for at least 4 hours after use. The four other products were shown to decrease the sebum level for at least 6 hours after a single application.

### Example 10

### Evaluation Of Two Products Containing Salicylic Acid And Hydrolysed Milk Protein

### Design

A trial was performed on different groups of 50 teenagers, of both sexes, between 12 and 19 years old suffering from impurities/spots/pimples. One of these groups tested a gel wash (Example 3) and another group tested a cream scrub (Example 5) in their normal condition of use. After 3 days use, the volunteers were contacted by phone and were asked to answer to questions regarding the safety, efficacy and cosmetic acceptability of the products. Likewise questions were asked by phone after 2 weeks use.

### Results

Below is the mean of the volunteer's response to some of the questions after 3 days and 14 days use :

Regarding the Cream Scrub, 64% of the volunteers were overall very satisfied or satisfied from day 3 and even more after 14 days with 74% of the panel. This satisfaction is mainly due to better skin condition from day 3 (slightly to significantly better 64%) including pore looking smaller from Day 3 (strongly and largely agree 60%) and due to the effectiveness to combat pimples from day 3 (strongly and largely agree 68%) and to prevent from new pimples at day 14 (strongly and largely agree 64%). Respectively 58% and 60% of the volunteers strongly or largely agree that they have less pimples and less blackheads when they use it regularly. In addition to this, their skin is softer and smoother from day 3 (strongly and largely agree respectively, 80% and 72%) and even more after 2 weeks (strongly and largely agree respectively, 86% and 80%).
This product also has a recognised action on the sebum level because 70% of the volunteers strongly and largely agree that it decreases shine after use and after 14 days, it delays the return of greasiness after application (strongly and largely agree 66%).

Regarding the Gel Wash, 69% of the volunteers were overall very satisfied or satisfied from day 3 and after 14 days with 60% of the panel. Again this satisfaction is mainly due to better skin condition from day 3 (slightly to significantly better 63%) and due to the effectiveness to combat pimples from day 3 (strongly and largely agree 63%). In addition to this, their skin is also softer and smoother from day 3 (strongly and largely agree respectively, 78% and 72%) and continues for 2 weeks (strongly and largely agree respectively, 67% and 71%). Furthermore, the product reduces skin shine right after use as assessed from day 3 (strongly and largely agree respectively, 66%) and after 14 days, it delays the return of greasiness after application (strongly and largely agree 67%).

### Conclusion

Both products containing salicylic acid and HMP clearly act simultaneously:
- on sebum by showing a shine reduction and delaying the return of greasiness
- on pimples and blackheads.
These 2 products containing the active combination are clearly appreciated for their overall efficacy and their positive action on skin condition.

## Claims

1. A skincare composition with a pH in the range 2.5-6.0, suitable for topical application to the skin, the composition comprising 0.1-5% by weight salicylic acid or a salt thereof and hydrolysed milk protein, wherein the ratio of salicylic acid or salt thereof to hydrolysed milk protein is in the range from 2:1 to 15:1 parts by weight.

2. A composition as claimed in Claim 1, which comprises salicylic acid.

3. A composition as claimed in Claim 2, wherein the concentration of salicylic acid is at least 1% by weight.

4. A composition as claimed in Claim 2, wherein the concentration of salicylic acid is less than 3% by weight.

5. A composition as claimed in Claim 2, wherein the concentration of salicylic acid is in the range from 1 % to 3% by weight.

6. A composition as claimed in Claim 1. wherein the concentration of hydrolysed milk protein is at least 0.05% by weight, and most preferably at least 0.1% by weight.

7. A composition as claimed in Claim 1, wherein the concentration of hydrolysed milk protein is less than 1% by weight

8. A composition as claimed in Claim 1, wherein the concentration of hydrolysed milk protein is in the range from 0.1 % to 1.0% by weight.

9. A composition as claimed in claim 1, wherein the concentration of salicylic acid is in the range from 0.5 to 4% by weight, more preferably from 0.5 to 2% by weight and the concentration of hydrolysed milk protein is in the range from 0.08 to 2%, more preferably from 0.1 to 0.5 % by weight.

10. A composition as claimed in any preceding claim, wherein the ratio of salicylic acid or salt thereof to hydrolysed milk protein is in the range from 5:1 to 12:1 parts by weight.

11. A composition as claimed in any preceding claim, wherein the pH is in the range from 2.5 to 4.0.

12. A composition as claimed in any preceding claim further comprising one or more further topically active skincare agents selected from an antimicrobial or anti-bacterial compound, an anti-viral compound, an anti-fungal compound, an anti-inflammatory compound and an anthelmintic compound.

13. A composition as claimed in claim 12 wherein the anti-bacterial agent is a peroxide anti-bacterial agent

14. A composition as claimed in any preceding claim, which has the form of an aqueous or oily solution or dispersion or emulsion or a gel.

15. A composition as claimed In Claim 14, which is In the form of an emulsion.

16. A composition as claimed in Claim 15, wherein the emulsion is an oil-in-water emulsion.

17. A composition as claimed in Claim 15, wherein the emulsion is a water-in-oil emulsion.

18. A composition as claimed in claim 14, which is in the form of an aqueous gel.

19. A composition as claimed in any preceding claim, which further comprises a gelling and/or a thickening agent.

20. A composition as claimed in Claim 19, wherein the gelling agent is a copolymer of acryloyl dimethyl tauric acid or a salt thereof.

21. A composition as claimed in any preceding claim, which comprises an aqueous solvent system.

22. A composition as claimed in Claim 21, wherein the solvent system is a mixed solvent system comprising water in admixture with a co-solvent.

23. A composition as claimed in Claim 22, wherein the co-solvent is an alcohol.

24. A composition as claimed in any preceding claim, which comprises one or more excipients selected from the group consisting of emulsifiers, emollients, lipids, humectants or moisturisers, binders, conditioning agents, emulsion stabilising salts, preservatives, chelating agents or sequestering agents, abrasives, pH adjusters, surfactants, perfumes and colourings.

25. An article comprising a fibrous substrate impregnated with a skincare composition with a pH in the range 2.5-6.0, suitable for topical application to the skin, the composition comprising 0.1-5% by weight salicylic acid or a salt thereof and hydrolysed milk protein, wherein the ratio of salicylic acid or salt thereof to hydrolysed milk protein is in the range from 2:1 to 15:1 parts by weight.

26. An article as claimed in claim 25, wherein the fibrous material is impregnated with the skincare composition in an amount in the range from 10 to 30% by weight, preferably from 15 to 25% by weight and most preferably from 18 to 22% by weight of the fibrous material.

27. An article as claimed in either one of claims 25 or 26 comprising cellulose or cotton fibres or a mixture thereof.

28. Use of hydrolysed milk protein in the preparation of a composition comprising 0.1-5% by weight salicylic acid or a salt thereof and having a pH in the range 2.5-6.0, wherein the ratio of salicylic acid or salt thereof to hydrolysed milk protein is in the range from 2:1 to 15:1 parts by weight for the prophylactic or remedial medical treatment of acne by topical application of the composition to the skin.

29. Use of hydrolysed milk protein in a composition comprising 0.1-5% by weight salicylic acid or a salt thereof and having a pH in the range 2.5-6.0, wherein the ratio of salicylic acid or salt thereof to hydrolysed milk protein is in the range from 2:1 to 15:1 parts by weight.

30. The use as claimed in either one of claims 28 or 29 wherein salicylic acid or a salt thereof and hydrolysed milk protein are the sole active ingredients in the composition.

## Patentansprüche

1. Hautpflegezusammensetzung mit einem pH-Wert im Bereich von 2,5-6,0, geeignet zur topischen Aufbringung auf die Haut, wobei die Zusammensetzung 0,1-5 Gew.-% Salicylsäure oder eines Salzes davon und hydrolysiertes Milchprotein umfasst, wobei das Verhältnis von Salicylsäure oder Salz davon zu hydrolysiertem Milchprotein im Bereich von 2:1 bis 15:1 Gewichtsteilen liegt.

2. Zusammensetzung nach Anspruch 1, die Salicylsäure umfasst.

3. Zusammensetzung nach Anspruch 2, wobei die Konzentration von Salicylsäure mindestens 1 Gew.-% beträgt.

4. Zusammensetzung nach Anspruch 2, wobei die Konzentration von Salicylsäure weniger als 3 Gew.-% beträgt.

5. Zusammensetzung nach Anspruch 2, wobei die Konzentration von Salicylsäure im Bereich von 1 bis 3 Gew.-% liegt.

6. Zusammensetzung nach Anspruch 1, wobei die Konzentration von hydrolysiertem Milchprotein mindestens 0,05 Gew.-% und am meisten bevorzugt mindestens 0,1 Gew.-% beträgt.

7. Zusammensetzung nach Anspruch 1, wobei die Konzentration von hydrolysiertem Milchprotein weniger als 1 Gew.-% beträgt.

8. Zusammensetzung nach Anspruch 1, wobei die Konzentration von hydrolysiertem Milchprotein im Bereich von 0,1 bis 1,0 Gew.-% liegt.

9. Zusammensetzung nach Anspruch 1, wobei die Konzentration von Salicylsäure im Bereich von 0,5 bis 4 Gew.-%, stärker bevorzugt von 0,5 bis 2 Gew.-% und die Konzentration von hydrolysiertem Milchprotein im Bereich von 0,08 bis 2 Gew.-%, stärker bevorzugt von 0,1 bis 0,5 Gew.-% liegt.

10. Zusammensetzung nach einem der vorangehenden Ansprüche, wobei das Verhältnis von Salicylsäure oder Salz davon zu hydrolysiertem Milchprotein im Bereich von 5:1 bis 12:1 gewichtsteilen liegt.

11. Zusammensetzung nach einem der vorangehenden Ansprüche, wobei der pH-Wert im Bereich von 2,5 bis 4,0 liegt.

12. Zusammensetzung nach einem der vorangehenden Ansprüche, ferner umfassend ein oder mehrere weitere topisch wirksame Hautpflegemittel, ausgewählt aus einer antimikrobiellen oder antibakteriellen Verbindung, einer antiviralen Verbindung, einer antifungalen Verbindung, einer entzündungshemmenden Verbindung und einer anthelmintischen Verbindung.

13. Zusammensetzung nach Anspruch 12, wobei das antibakterielle Mittel ein antibakerielles Peroxidmittel ist.

14. Zusammensetzung nach einem der vorangehenden Ansprüche, die die Form einer wässrigen oder öligen Lösung oder Dispersion oder Emulsion oder eines Gels aufweist.

15. Zusammensetzung nach Anspruch 14, die in Form einer Emulsion vorliegt.

16. Zusammensetzung nach Anspruch 15, wobei die Emulsion eine Öl-in-Wasser-Emulsion ist.

17. Zusammensetzung nach Anspruch 15, wobei die Emulsion eine Wasser-in-Öl-Emulsion ist.

18. Zusammensetzung nach Anspruch 14, die in Form eines wässrigen Gels vorliegt.

19. Zusammensetzung nach einem der vorangehenden Ansprüche, die ferner einen Gelbildner und/oder ein Eindickungsmittel umfasst.

20. Zusammensetzung nach Anspruch 19, wobei der Gelbildner ein Copolymer von Acryloyldimethyltaurinsäure oder einem Salz davon ist.

21. Zusammensetzung nach einem der vorangehenden Ansprüche, die ein wässriges Lösungsmittelsystem umfasst.

22. Zusammensetzung nach Anspruch 21, wobei das Lösungsmittelsystem ein gemischtes Lösungsmittelsystem ist, das Wasser in gemisch mit einem Verschnittmittel umfasst.

23. Zusammensetzung nach Anspruch 22, wobei das Verschnittmittel ein Alkohol ist.

24. Zusammensetzung nach einem der vorangehenden Ansprüche, die ein oder mehrere Exzipienten umfasst, ausgewählt aus der Gruppe, bestehend aus Emulgatoren, Weichmachern, Lipiden, Feuchtmitteln oder feuchtigkeitsspendenden Mitteln, Bindemitteln, Conditionern, elumlsionsstabilisierenden Salzen, Konservierungsmitteln, Chelatbildnern oder Maskierungsmitteln, Schleifmitteln, Ph-einstellenden Mitteln, oberflächenaktiven Mitteln, Parfums und Farbmitteln.

25. Gegenstand, umfassend ein faserartiges Substrat, imprägniert mit einer Hautpflegezusammensetzung mit einem pH-Wert im Bereich von 2,5-6,0, geeignet zur topischen Aufbringung auf die Haut, wobei die Zusammensetzung 0,1-5 Gew.-% Salicylsäure oder ein Salz davon und hydrolysiertes Milchprotein umfasst, wobei das Verhältnis von Salicylsäure oder Salz davon zu hydrolysiertem Milchprotein im Bereich von 2:1 bis 15:1 Gewichtsteilen liegt.

26. Gegenstand nach Anspruch 25, wobei das faserartige Material mit der Hautpflegezusammensetzung in einer Menge im Bereich von 10 bis 30 Gew.-%, vorzugsweise 15 bis 25 Gew.-% und am meisten bevorzugt 18 bis 22 Gew.-% des faserartigen Materials imprägniert ist.

27. Gegenstand nach einem der Ansprüche 25 oder 26, umfassend Cellulose- oder Baumwollfasern oder ein Gemisch davon.

28. Verwendung von hydrolysiertem Milchprotein in einer Zusammensetzung, umfassend 0,1-5 Gew.-% Salicylsäure oder eines Salzes davon und aufweisend einen pH-Wert im Bereich von 2,5-6,0, wobei das Verhältnis von Salicylsäure oder Salz davon zu hydrolysiertem Milchprotein im Bereich von 2:1 bis 15:1 Gewichtsteilen liegt, zur prophylaktischen oder abhelfenden medizinischen Behandlung von Akne durch topische Aufbringung der Zusammensetzung auf die Haut.

29. Verwendung von hydrolysiertem Milchprotein bei der Herstellung einer Zusammensetzung, umfassend 0,1-5 Gew.-% Salicylsäure oder eines Salzes davon und aufweisend einen pH-Wert im Bereich von 2,5-6,0, wobei das Verhältnis von Salicylsäure oder Salz davon zu hydrolysiertem Milchprotein im Bereich von 2:1 bis 15:1 Gewichtsteilen liegt.

30. Verwendung nach einem der Ansprüche 28 oder 29, wobei Salicylsäure oder ein Salz davon und hydrolysiertes Milchprotein die einzigen Wirkstoffe in der Zusammensetzung sind.

## Revendications

1. Composition pour soins cutanés ayant un pH compris entre 2,5 et 6,0, convenable pour l'application topique à la peau, la composition comprenant 0,1 à 5 % en poids d'acide salicylique ou d'un de ses sels et de la protéine de lait hydrolysée, dans laquelle le rapport de l'acide salicylique ou de son sel à la protéine de lait hydrolysée est de 2:1 à 15:1 parties en poids.

2. Composition suivant la revendication 1, qui comprend de l'acide salicylique.

3. Composition suivant la revendication 2, dans laquelle la concentration en acide salicylique est d'au moins 1 % en poids.

4. Composition suivant la revendication 2, dans laquelle la concentration d'acide salicylique est inférieure à 3 % en poids.

5. Composition suivant la revendication 2, dans laquelle la concentration d'acide salicylique est de 1 % à 3 % en poids.

6. Composition suivant la revendication 1, dans laquelle la concentration de protéine de lait hydrolysée est d'au moins 0,05 % en poids et de préférence d'au moins 0,1 % en poids.

7. Composition suivant la revendication 1, dans laquelle la concentration de protéine de lait hydrolysée est inférieure à 1 % en poids.

8. Composition suivant la revendication 1, dans laquelle la concentration de protéine de lait hydrolysée est de 0,1 % à 1,0 % en poids.

9. Composition suivant la revendication 1, dans laquelle la concentration d'acide salicylique est de 0,5 à 4 % en poids, plus avantageusement de 0,5 à 2 % en poids, et la concentration de protéine de lait hydrolysée est de 0,08 à 2 %, plus avantageusement de 0,1 à 0,5 %, en poids.

10. Composition suivant l'une quelconque des revendications précédentes, dans laquelle le rapport de l'acide salicylique ou de son sel à la protéine de lait hydrolysée est de 5:1 à 12:1 parties en poids.

11. Composition suivant l'une quelconque des revendications précédentes, dans laquelle le pH est de 2,5 à 4,0.

12. Composition suivant l'une quelconque des revendications précédentes, comprenant en outre un ou plusieurs agents topiquement actifs supplémentaires pour soins cutanés, choisis entre un composé antimicrobien ou antibactérien, un composé antiviral, un composé antifongique, un composé anti-inflammatoire et un composé anthelmintique.

13. Composition suivant la revendication 12, dans laquelle l'agent antibactérien est un agent antibactérien du type peroxyde.

14. Composition suivant l'une quelconque des revendications précédentes, qui est sous forme d'une solution, dispersion ou émulsion aqueuse ou huileuse ou d'un gel.

15. Composition suivant la revendication 14, qui est sous forme d'une émulsion.

16. Composition suivant la revendication 15, dans laquelle l'émulsion est une émulsion huile-dans-eau.

17. Composition suivant la revendication 15, dans laquelle l'émulsion est une émulsion eau-dans-huile.

18. Composition suivant la revendication 14, qui est sous forme d'un gel aqueux.

19. Composition suivant l'une quelconque des revendications précédentes, qui comprend en outre un agent gélifiant et/ou épaississant.

20. Composition suivant la revendication 19, dans laquelle l'agent gélifiant est un copolymère d'acide acryloyldiméthyltaurique ou d'un de ses sels.

21. Composition suivant l'une quelconque des revendications précédentes, qui comprend un système de solvant aqueux.

22. Composition suivant la revendication 21, dans laquelle le système de solvant est un système de solvant mixte comprenant de l'eau en mélange avec un cosolvant.

23. Composition suivant la revendication 22, dans laquelle le cosolvant est un alcool.

24. Composition suivant l'une quelconque des revendications précédentes, qui comprend un ou plusieurs excipients choisis dans le groupe consistant en des émulsionnants, des émollients, des lipides, des agents hydratants ou humidifiants, des liants, des agents conditionnants, des sels stabilisateurs d'émulsion, des conservateurs, des agents chélatants ou agents séquestrants, des abrasifs, des agents d'ajustement du pH, des agents tensioactifs, des parfums et des colorants.

25. Article comprenant un substrat fibreux imprégné d'une composition pour soins cutanés ayant un pH compris entre 2,5 et 6,0, convenable pour l'application topique à la peau, la composition comprenant 0,1 à 5 % en poids d'acide salicylique ou d'un de ses sels et de la protéine de lait hydrolysée, le rapport de l'acide salicylique ou de son sel à la protéine de lait hydrolysée étant de 2:1 à 15:1 parties en poids.

26. Article suivant la revendication 25, dans lequel la matière fibreuse est imprégnée de la composition pour soins cutanés en une quantité de 10 à 30 % en poids, avantageusement de 15 à 25 % en poids et de préférence de 18 à 22 % en poids de la matière fibreuse.

27. Article suivant l'une des revendications 25 et 26, comprenant des fibres de cellulose ou de coton ou un de leurs mélanges.

28. Utilisation de la protéine de lait hydrolysée dans la préparation d'une composition comprenant 0,1 à 5 % en poids d'acide salicylique ou d'un de ses sels et ayant un pH de 2,5 à 6,0, le rapport de l'acide salicylique ou de son sel à la protéine de lait hydrolysée étant de 2:1 à 15:2 parties en poids, pour le traitement médical prophylactique ou curatif de l'acné par application topique de la composition à la peau.

29. Utilisation de la protéine de lait hydrolysée dans une composition comprenant 0,1 à 5 % en poids d'acide salicylique ou d'un de ses sels et ayant un pH de 2,5 à 6,0, dans laquelle le rapport de l'acide salicylique ou de son sel à la protéine de lait hydrolysée est de 2:1 à 15:1 parties en poids.

30. Utilisation suivant l'une des revendications 28 et 29, dans laquelle l'acide salicylique ou un de ses sels et la protéine der lait hydrolysée sont les seuls ingrédients actifs présents dans la composition.
